Europäisches Patentamt

European Patent Office

Office européen des brevets

(19)

(11) Veröffentlichungsnummer: **0 215 375**
**B1**

(12) **EUROPÄISCHE PATENTSCHRIFT**

(45) Veröffentlichungstag der Patentschrift: **12.12.90**

(51) Int. Cl.⁵: **A 61 N 1/05**

(21) Anmeldenummer: **86112173.9**

(22) Anmeldetag: **03.09.86**

(54) **Herzschrittmacherelektrode.**

(30) Priorität: **09.09.85 DE 3532084**

(43) Veröffentlichungstag der Anmeldung:
**25.03.87 Patentblatt 87/13**

(45) Bekanntmachung des Hinweises auf die
Patenterteilung:
**12.12.90 Patentblatt 90/50**

(84) Benannte Vertragsstaaten:
**DE FR GB IT NL SE**

(56) Entgegenhaltungen:
**EP-A-0 032 356**
**US-A-3 977 411**

(73) Patentinhaber: **Siemens Elema AB**
**Röntgenvägen 2**
**S-171 95 Solna 1 (SE)**
(84) **SE**

(73) Patentinhaber: **Siemens Aktiengesellschaft**
**Wittelsbacherplatz 2**
**D-8000 München 2 (DE)**
(84) **DE FR GB IT NL**

(72) Erfinder: **Lekholm, Anders, Dr.**
**Gnejsvaegen 4**
**S-161 39 Bromma (SE)**

Courier Press, Leamington Spa, England.

**Beschreibung**

Die Erfindung betrifft eine Herzschrittmacherelektrode mit mindestens einem elektrischen Leiter, der von einer gegen Körperflüssigkeit inerten Isolierhülle umgeben ist und zumindest am distalen Ende mit einem leitfähigen Elektrodenkopf versehen ist. Derartige Herzschrittmacherelektroden können sowohl für die Stimulierung des Myokardiums als auch zum Detektieren von Herz-Potentialen verwendet werden. Um die notwendige Stimulierungsenergie möglichst klein zu halten, soll die Stromdichte so gross wie möglich sein, was sich am einfachsten durch eine möglichst kleine Oberfläche des leitfähigen Elektrodenkopfes erreichen lässt. Werden derartige Elektroden zum Abfühlen der QRS-Potentiale verwendet, haben sie bei glatter Elektrodenkopfoberfläche eine relativ hohe Quellenimpedanz in der Grössenordnung 10 bis 20 kohm. Da die Eingangsimpedanz der nachfolgenden Verstärker oft in der gleichen Grössenordnung liegt, werden diese Herzsignale in der Eingangsstufe stark gedämpft. Die Abfühlcharakteristika können am einfachsten dadurch verbessert werden, dass die leitfähige Oberfläche des Elektrodenkopfes vergrössert wird. Die Anforderungen an die Grösse der Elektrodenoberfläche sind für das Stimulieren und das Abfühlen genau gegensätzlich, weshalb eine Optimierung vorgenommen werden muss.

Dieses Problem gegensätzlicher Anforderungen an den Elektrodenkopf kann auch auf andere Art und Weise gelöst werden, beispielsweise dadurch, dass die Oberfläche des Elektrodenkopfes mikroporös gemacht wird. Dadurch wird der Strom bei der Stimulierung konzentriert und damit die gewünschte hohe Stromdichte erhalten, während die Grenzfläche Elektrodenkopf/Elektrolyt gross genug ist, um eine niedrige Quellenimpedanz beim Abfühlen von Herzsignalen zu erhalten. Derartige Elektroden sind beispielsweise aus dem Siemens Prospekt über Endokardelektroden mit einem Elektrodenkopf aus glasartigem Kohlenstoff vom März 1984 (A91003-M3372-L745-02-4J00) bekannt.

Weiterhin ist aus der EP—A—0 032 356 oder der US—A—3,977,411 eine Herzschrittmacherelektrode zum gleichzeitigen Stimulieren bzw. Abfühlen bekannt. Bei dieser Herzschrittmacherelektrode sind zwei separate, durch eine Isolierschicht voneinander getrennte Flächen unterschiedlicher Grösse vorgesehen, wobei die grössere Fläche als Sensingfläche dient und über einen relativ hochohmigen Widerstand an den gemeinsamen Leiter angeschlossen ist, um beim Stimulieren praktisch nicht zum Stromtransport beizutragen. Die Leitfähigkeit der Stimulierungs- und Sensingflächen sind auf die Fläche bezogen gleich.

Der vorliegenden Erfindung liegt die Aufgabe zugrunde, eine Elektrode dieser Art derart auszubilden, dass sie trotz guter Stimulierungs- als auch Sensingeigenschaften grosse Freiheiten bei der räumlichen Gestaltung des Elektrodenkopfes lässt und in der Herstellung einfach ist.

Diese Aufgabe ist erfindungsgemäss dadurch gelöst, dass die beiden Flächen für Stimulierung und Sensing unterschiedliche Impedanz aufweisen derart, dass die Impedanz der Sensingfläche so gross ist, dass sich beim Stimulieren der Strom auf die Stimulationsfläche konzentriert, beim Abfühlen hingegen beide Flächen wirksam sind. Im Gegensatz zu den beispielsweise aus der US—A—3,977,411 bekannten Herzschrittmacherelektrode ist bei der vorliegenden Elektrode keine galvanische Trennung zwischen den Flächen notwendig und auch kein zusätzlicher Widerstand zwischen einer dieser Flächen und dem Elektrodenleiter.

Die beiden Flächen unterschiedlicher Impedanz können auf einfacher Weise durch Materialien unterschiedlicher Leifähigkeit gebildet werden. So kann beispielsweise der Elektrodenkopf eine Spitze kleiner Oberfläche mit guter Leifähigkeit für die Stimulierung aufweisen. An diese Spitze schliesst sich dann eine grössere Oberfläche mit niedrigerer Leitfähigkeit an. Beim Stimulieren konzentriert sich daher der Strom auf die Spitze, da die Impedanz zum Gewebe relativ niedrig ist, beispielsweise 500—1000 Ohm. Beim Abfühlen dagegen ist die gesamte Oberfläche des Elektrodenkopfes wirksam, da die Quellenimpedanz des Elektrodenkopfes mehrere kOhm betragen kann, ohne die Abfühleigenschaften nennenswert zu beeinflussen. Anders ausgedrückt macht die Erfindung von der Erkenntnis Gebrauch, dass an der Grenzfläche zwischen Elektrodenkopfoberfläche und Elektrolyt ein grosser Impedanzunterschied besteht je nach dem, ob es sich um kleine oder grosse Signale handelt.

Anstelle unterschiedlicher Materialien lässt sich die unterschiedliche Impedanz auch durch Oberflächenbehandlung des gleichen Materiales erzielen. So kann die Fläche hoher Impedanz beispielsweise eine glatte Oberfläche aufweisen, die Fläche niedriger Impedanz hingegen eine mikroporöse Oberfläche. In einer vorteilhaften Weiterbildung der Erfindung ist vorgesehen, dass die Stimulationsfläche aus Metall- bzw. Metallverbindungen und die Sensingfläche aus leitfähigem, gegen Körperflüssigkeit inertem Kunststoff bestehen. Wie bereits ausgeführt, kann die Spitze des Elektrodenkopfes aus Material mit niedriger Impedanz bestehen und für die Stimulierung vorgesehen sein. Es ist jedoch vorteilhaft auch möglich, die Fläche niedriger Impedanz, d.h. die Stimulationsfläche, in mehrere Teilbereiche einzuteilen und über die gesamte Oberfläche des Elektrodenkopfes oder ein Teil dieser Fläche gleichmässig oder auch ungleichmässig zu verteilen. So können die Bereich niedriger Impedanz ringförmig über den Elektrodenkopf verteilt sein. Ebenso ist es möglich, dass die Bereiche streifenförmig verteilt sind oder ein Käfigmuster bilden. In jedem Fall ist die gesamte Oberfläche des Elektrodenkopfes grösser und durch die Verteilung der für die Stimulierung massgeblichen Bereiche über diese grosse Oberfläche ergibt sich der Vorteil, dass mit Sicherheit einige dieser Bereiche Kontakt mit gut leitfähigem Körpergewebe geben und insgesamt der Schwellwert niedrig gehalten werden kann.

Insbesondere bei der Verwendung leitfähigen Kunststoffes ergibt sich ein weiterer Vorteil dadurch, dass die aus diesem Kunststoff gebildete Fläche für das Abfühlen von Herzaktivitäten gleichzeitig durch besondere Formgebung noch zur Fixierung des Elektrodenkopfes im Trabekelwerk dienen kann. Diese so gebildeten Halteorgane können in Form von Borsten oder Flossen oder als Kragen ausgebildet sein und eine relativ grosse Oberfläche aufweisen, durch die die Abfühlempfindlichkeit stark erhöht werden kann. Die Halteorgane können zum passiven, aber auch zum aktiven Fixieren vorgesehen sein.

In einer weiteren Ausgestaltung der Erfindung ist es ebenso denkbar, dass die Impedanz der Halteorgane grösser ist als die der Spitze des Elektrodenkopfes und dass somit die Halteorgane zur Stimulierung dienen. An Hand von sechs Figuren werden im folgenden vier Ausführungsbeispiele der Erfindung näher beschrieben und erläutert. Dabei zeigen:

Fig. 1 eine erste Ausführungsform der erfindungsgemässen Elektrode,

Fig. 2 und 3 zwei Ansichten einer Ausführungsform mit flossenförmigen Halteorganen,

Fig. 4 eine weitere Ausführungsform mit ringförmig verteilten Bereichen hoher Leitfähigkeit und

Fig. 5 und 6 schliesslich erneut eine Ausführungsform mit Halteorganen.

Sämtliche Figuren zeigen lediglich in einer schematischen Darstellung und nicht unbedingt massstabsgetreu das distale Ende einer Elektrodenleitung.

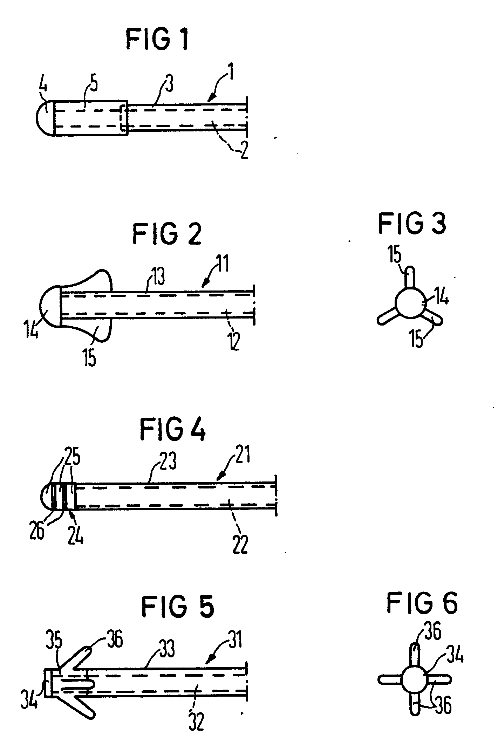

In Figur 1 ist die Elektrodenleitung mit 1 bezeichnet. Gestrichelt ist ein elektrischer Leiter 2 angedeutet, der von einer Isolierhülle 3 umgeben ist. Am distalen Ende ist der Leiter 2 mit einer Spitze 4 aus glattem oder mikroporösem Metall, einer Metallverbindung oder auch Kohlenstoff verbunden. Die Spitze 4 weist eine niedrige Impedanz auf und eignet sich hervorragend zur Stimulierung. Die Oberfläche dieser Spitze soll möglichst klein sein, um eine genügend hohe Stromdichte zu erzielen und kann beispielsweise 10 mm² betragen. An diese Spitze schliesst sich ein elektrisch mit dieser verbundener Ring 5 an, der beispielsweise aus einem leitfähigem Elastomer oder einem Metall geringer Leitfähigkeit bestehen kann. Ebenso kommen Metallverbindungen oder Keramikverbindungen in Frage. Die Oberfläche dieses Ringes ist grösser als die der Spitze 4. Durch die geringere Leitfähigkeit des Ringes 5 fliesst während des Stimulierens praktisch kein Strom über diese Oberfläche. Die Leitfähigkeit dieses Ringes ist jedoch gross genug, um beim Abfühlen von herzeigenen Potentialen diese Signale zusammen mit der Spitze 4 aufzunehmen.

Die Figuren 2 und 3 zeigen eine weitere Herzschrittmacherelektrode 11, wiederum mit einem Leiter 12 und einer diesen umgebenden Isolierhülle 13. Anschliessend an eine Spitze 14 sind elektrisch mit dieser verbunden drei flossenähnliche Vorsprünge 15 vorgesehen, die aus einem weniger gut leitenden Material als die Spitze 14 gebildet sind. Die Flossen 15 können wiederum aus einem leitfähigen Elastomer bestehen und dienen neben dem Vergrösser der Oberfläche des Elektrodenkopfes zum Abfühlen von Herzpotentialen gleichzeitig auch zur passiven Fixierung des Elektrodenkopfes. Figur 3 zeigt die Elektrode von vorne.

In Figur 4 ist eine weitere Herzschrittmacherelektrode 21 mit einem elektrischen Leiter 22 und einer Isolierhülle 23 dargestellt, an derem distalen Ende sich ein Elektrodenkopf 24 befindet. Die Fläche dieses Elektrodenkopfes besteht überwiegend aus Bereichen 25 mit geringerer Leitfähigkeit. Ringförmig sind in diesem Ausführungsbeispiel zwei Bereiche 26 vorgesehen, die eine hohe Leitfähigkeit haben und die zur Stimulierung dienen.

In den Figuren 5 und 6 ist eine Herzschrittmacherelektrode 31 mit einem Leiter 32 und einer Isolierhülle 33 dargestellt, die am distalen Ende mit einer platten Spitze 34 aus Material niedriger Impedanz versehen ist, an die sich ein Ring aus einem leitfähigem Elastomer 35 anschliesst, von dem borstenähnliche passive Halteorgane 36 aus ebenfalls leitfähigem Material hervorstehen. Figur 6 zeigt die Herzschrittmacherelektrode wieder von vorne.

| | Bezugszeichenliste |
|---|---|
| 1 | Elektrodenleitung |
| 2, 12, 22, 32 | elektrisscher Leiter |
| 3, 13, 23, 33 | Isolierhülle |
| 4, 14, | Spitze |
| 5 | Ring |
| 11, 21, 31 | Herzschrittmacherelektrode |
| 15 | Flossen |
| 24 | Elektrodenkopf |
| 25 | Bereich geringer Leitfähigkeit |
| 26 | Bereich hoher Leitfähigkeit |
| 34 | platte Spitze |
| 35 | Ring aus leitfähigem Elastomer |
| 36 | Halteorgane |

**Patentansprüche**

1. Herzschrittmacherelektrode mit mindestens einem elektrischen Leiter (2, 12, 22, 32), der von einer gegen Körperflüssigkeit inerten Isolierhülle (3, 13, 23, 33) umgeben ist und am distalen Ende mit einem Elektrodenkopf versehen ist, der eine Stimulationsfläche (4, 14, 26, 34) und eine separate Sensingfläche (5, 15, 25, 35, 36) aufweist, dadurch gekennzeichnet, dass beide Flächen direkt galvanisch mit dem herznahen Ende des elektrischen Leiters verbunden sind, und die beiden Flächen unterschiedliche Impedanz aufweisen derart, dass die Impedanz der Sensingfläche (5, 15, 25, 35, 36) so gross ist, dass sich beim Stimulieren der Strom auf die Stimulationsfläche (4, 14, 26, 34) konzentriert, beim Abfühlen hingegen beide Flächen wirksam sind.

2. Herzschrittmacher nach Anspruch 1, dadurch gekennzeichnet, dass die unterschiedliche Impedanz der Stimulations- bzw. Sensingfläche durch Materialien unterschiedlicher Leitfähigkeit gebildet ist.

3. Herzschrittmacher nach Anspruch 1, dadurch gekennzeichnet, dass die unterschiedliche Impedanz der Stimulations- bzw. Sensingfläche durch unterschiedliche Oberflächenbehandlung dieser Flächen erzeugt ist.

4. Herzschrittmacher nach Anspruch 3, dadurch gekennzeichnet, dass die Fläche niedriger Impedanz eine mikroporöse Oberfläche aufweist.

5. Herzschrittmacher nach Anspruch 2, dadurch gekennzeichnet, dass die Stimulationsfläche aus Metall oder einer Metallverbindung und die Sensingfläche aus leitfähigem, gegen Körperflüssigkeit inertem Kunststoff bestehen.

6. Herzschrittmacher nach einem der Ansprüche 1 bis 5, dadurch gekennzeichnet, dass die Spitze des Elektrodenkopfes die niedrigere Impedanz aufweist.

7. Herzschrittmacherelektrode nach einem der Ansprüche 1 bis 5, dadurch gekennzeichnet, dass die Stimulationsfläche (26) ringförmig über den Elektrodenkopf (24) verteilt ist.

8. Herzschrittmacherelektrode nach Anspruch 1 bis 5, dadurch gekennzeichnet, dass mehrere Flächen (25, 26) unterschiedlicher Impedanz über den Elektrodenkopf verteilt sind.

9. Herzschrittmacherelektrode nach Anspruch 8, dadurch gekennzeichnet, dass die Flächen (25, 26) unterschiedlicher Impedanz gleichmässig über den Elektrodenkopf verteilt sind.

10. Herzschrittmacherelektrode nach Anspruch 1 bis 5 mit Halteorganen (15, 36) zum Fixieren des Elektrodenkopfes am Herzen, dadurch gekennzeichnet, dass die Oberfläche dieser Halteorgane (15, 36) eine der beiden Flächen unterschiedlicher Impedanz bildet.

11. Herzschrittmacher nach Anspruch 10, wobei die Halteorgane (15, 36) in bekannter Weise zum passiven Fixieren im Trabekelwerk des Herzens ausgebildet sind.

12. Herzschrittmacher nach einem der Ansprüche 10 oder 11, dadurch gekennzeichnet, dass die Oberfläche der Halteorgane (15, 36) als Sensingfläche dient.

13. Herzschrittmacherelektrode nach Anspruch 12, dadurch gekennzeichnet, dass die Halteorgane (15, 36) aus leitfähigem Kunststoff bestehen.

14. Herzschrittmacherelektrode nach einem der vorangehenden Ansprüche, dadurch gekennzeichnet, dass die Gesamtfläche mit hoher Impedanz grösser ist als die Gesamtfläche niedriger Impedanz.

## Revendications

1. Electrode pour stimulateur cardiaque comportant au moins un conducteur électrique (2, 12, 22, 32), qui est entouré par une gaine isolante (3, 13, 23, 33) inerte vis-à-vis d'un liquide corporel et est pourvu, au niveau de son extrémité distale, d'une tête d'électrode qui possède une surface de stimulation (4, 14, 26, 34) et une surface distincte de détection (5, 15, 25, 35, 36), caractérisé par le fait que ces deux surfaces sont reliées directement, par voie galvanique, à l'extrémité, plus proche du coeur, du conducteur électrique, que les deux surfaces possèdent des impédances différentes telles que l'impédance de la surface de détection (5, 15, 5, 35, 36) est suffisamment élevée pour que, lors de la stimulation, le courant soit concentré sur la surface de stimulation (4, 14, 26, 34), tandis que lors de la détection, ces deux surfaces sont actives.

2. Stimulateur cardiaque suivant la revendication 1, caractérisé par le fait que l'impédance différente de la surface de stimulation et de la surface de détection est formée par des matériaux possédant des conductivités différentes.

3. Stimulateur cardiaque suivant la revendication 1, caractérisé par le fait que l'impédance différente de la surface de stimulation et de la surface de détection est obtenue au moyen d'un traitement superficiel différent de ces surfaces.

4. Stimulateur cardiaque suivant la revendication 3, caractérisé par le fait que la surface d'impédance inférieure possède une surface microporeuse.

5. Stimulateur cardiaque selon la revendication 2, caractérisé par le fait que la surface de stimulation est réalisée en un métal ou en un alliage métallique et que la surface de détection est réalisée en une matière plastique conductrice, inerte vis-à-vis d'un liquide corporel.

6. Stimulateur cardiaque selon l'une des revendications 1 à 5, caractérisé par le fait que la pointe de la tête d'électrode possède l'impédance la plus faible.

7. Electrode pour stimulateur cardiaque suivant l'une des revendications 1 à 5, caractérisée par le fait que la surface de stimulation (26) est répartie sous une forme annulaire autour de la tête d'électrode (24).

8. Electrode pour stimulateur cardiaque suivant les revendications 1 à 5, caractérisée par le fait que plusieurs surfaces (25, 26) possédant des impédances différentes sont réparties sur la tête d'électrode.

9. Electrode pour stimulateur cardiaque suivant la revendication 8, caractérisée par le fait que les surfaces (25, 26) possédant des impédances différentes sont réparties uniformément sur la tête d'électrode.

10. Electrode pour stimulateur cardiaque selon les revendications 1 à 5 comportant des éléments de retenue (15, 36) servant à fixer la tête d'électrode sur le coeur, caractérisée par le fait que la surface de ces éléments de maintien (15, 36) forme l'une des deux surfaces possédant des impédances différentes.

11. Stimulateur cardiaque suivant la revendication 10, dans lequel les éléments de retenue (15, 36) sont constitués, de façon connue, pour la fixation passive dans le réseau de ballonets musculaires du coeur (trabicula).

12. Stimulateur cardiaque suivant l'une des revendications 10 ou 11, caractérisé par le fait que la surface des éléments de retenue (15, 36) est utilisée comme surface de détection.

13. Electrode pour stimulateur cardiaque suivant la revendication 12, caractérisée par le fait que les éléments de retenue (15, 36) sont réalisés en une matière plastique conductrice.

14. Electrode pour stimulateur cardiaque suivant l'une des revendications précédentes, caractérisée par le fait que la totalité de la surface d'impédance élevée est supérieure à la totalité de la surface de faible impédance.

## Claims

1. Cardiac pacemaker electrode with at least one electrical coductor (2, 12, 22, 32), which is surrounded by an insulating covering (3, 13, 23, 33) inert with respect to body fluid and is provided at the distal end with an electrode head which has a stimulation surface (4, 14, 26, 34) and a separate sensing surface (5, 15, 25, 35, 36), characterized in that the two surfaces are directly connected galvanically to the end of the electrical conductor near the heart, and the two surfaces have different impedances such that the impedance of the sensing surface (5, 15, 25, 35, 36) is so large that during stimulation the current is concentrated on the stimulation surface (4, 14, 26, 34), whereas during sensing both surfaces are effective.

2. Cardiac pacemaker according to Claim 1, characterized in that the different impedances of the stimulation and sensing surface, respectively are formed by materials of different conductivity.

3. Cardiac pacemaker according to Claim 1, characterized in that the different impedances of the stimulation and sensing surfaces, respectively are generated by different surface treatment of these surfaces.

4. Cardiac pacemaker according to Claim 3, characterized in that the surface of lower impedance has a microporous surface.

5. Cardiac pacemaker according to Claim 2, characterized in that the stimulation surface consists of metal or of a metal compound, and the sensing surface consists of conductive plastic inert with respect to body fluid.

6. Cardiac pacemaker according to one of Claims 1 to 5, characterized in that the tip of the electrode head has the lower impedance.

7. Cardiac pacemaker electrode according to one of Claims 1 to 5, characterized in that the stimulation surface (26) is distributed annularly over the electrode head (24).

8. Cardiac pacemaker electrode according to Claims 1 to 5, characterized in that a plurality of surfaces (25, 26) of different impedances are distributed over the electrode head.

9. Cardiac pacemaker electrode according to Claim 8, characterized in that the surfaces (25, 26) of different impedances are distributed uniformly over the electrode head.

10. Cardiac pacemaker electrode according to Claims 1 to 5 having supporting elements (15, 36) for fixing the electrode head to the heart, characterized in that the surface of these supporting elements (15, 36) forms one of the two surfaces of different impedances.

11. Cardiac pacemaker according to Claim 10, the supporting elements (15, 36) being constructed in a known way for passive fixing in the trabecula carneae.

12. Cardiac pacemaker according to one of Claims 10 or 11, characterized in that the surface of the supporting elements (15, 36) serves as the sensing surface.

13. Cardiac pacemaker electrode according to Claim 12, characterized in that the supporting elements (15, 36) consist of conductive plastic.

14. Cardiac pacemaker electrode according to one of the perceding claims, characterized in that the total surface with high impedance is larger than the total surface of low impedance.

EP 0 215 375 B1

FIG 1

FIG 2

FIG 3

FIG 4

FIG 5

FIG 6

1